# EUROPEAN PATENT APPLICATION

(11) **EP 4 129 467 A1**
(43) Date of publication of application: **08.02.2023**
(21) Application number: 21776319.2
(22) Date of filing: 08.03.2021
(51) Int. Cl.: B01J 21/08, B01J 35/02, C07B 61/00, C07C 1/20, C07C 11/167

(54) **CATALYST AND METHOD FOR PRODUCING DIENE COMPOUND**

(30) Priority: 23.03.2020 JP 2020051785
(71) Applicant: SEKISUI CHEMICAL CO., LTD., Osaka-shi Osaka 530-8565 (JP)
(72) Inventor: TAKIZAWA, Koji, Tsukuba-shi, Ibaraki 300-4292 (JP); NISHIYAMA, Haruka, Tsukuba-shi, Ibaraki 300-4292 (JP)
(74) Representative: Ter Meer Steinmeister & Partner
(86) International application number: PCT/JP2021/008994
(87) International publication number: WO 2021/192960

(57) **Abstract**

A catalyst includes at least one element X selected from the group consisting of Groups 3 to 6 of the Periodic Table, and at least one element Z selected from the group consisting of Group 14 elements. The catalyst is flaky and has pores in a thickness direction. A catalyst that is capable of suppressing an overreaction to a polymer and producing a diene compound, particularly butadiene, at a high yield can be provided.

## Description

### Technical Field

The present invention relates to a catalyst and a method for producing a diene compound using the catalyst.

### Background Art

Butadienes such as 1,3-butadiene, which is a representative example of a diene compound, are used as a raw material of styrene-butadiene rubber (SBR) and the like. Conventionally, butadiene has been purified from the C4 fraction. The C4 fraction is a fraction produced as a by-product during naphtha cracking for which producing ethylene from petroleum. However, with the increase in shale gas use, the amount of oil that is used has decreased. As a result, the amount of butadiene obtained by naphtha cracking of petroleum is also decreasing. For this reason, alternative methods for producing diene compounds such as 1,3-butadiene are required.

In one method for producing 1,3-butadiene, 1,3-butadiene is produced by using ethanol as a raw material. For example, Patent Literature 1 to 3 disclose catalysts suitable for this reaction. For example, Production Example 1 of Patent Literature 1 discloses a catalyst in which Hf, Cu, and Zn are supported on silica, which is a porous carrier, and the examples of Patent Literature 2 disclose solid catalysts in which metals such as Zn, Zr, and alkaline earth metals are supported on a silica carrier. Further, the examples of Patent Literature 3 disclose a catalyst in which Ta, Zr, and Hf are each supported on silica as a carrier.

### Citation List

### Patent Literature

PTL1: JP 2019-43943 A
PTL2: JP 2020-998 A
PTL3: JP 2018-171587 A

### Summary of Invention

### Technical Problem

The butadiene synthesis catalysts described in Patent Literature 1 to 3 have a low butadiene yield in long-term continuous operation, and so there is room for improvement.

Accordingly, it is an object of the present invention to provide a catalyst capable of efficiently producing butadiene that is less susceptible to a decrease in the butadiene yield even in continuous operation for a long time.

### Solution to Problem

The present inventors have conducted intensive research to solve the above-described problem. As a result, it was found that the above-described problem is solved by a catalyst comprising predetermined elements and having a specific composite oxide structure, whereby the present invention was completed. That is, the invention has the following modes.
[1] A catalyst comprising a flaky particle comprising:
   at least one element X selected from the group consisting of Groups 3 to 6 of the Periodic Table; and
   at least one element Z selected from the group consisting of Group 14 elements, wherein
   the catalyst has pores in a thickness direction of the flaky particle.
[2] The catalyst according to [1], wherein the flaky particle has an average thickness of 50 to 600 nm.
[3] The catalyst according to [1] or [2], wherein the flaky particle is a composite oxide.
[4] The catalyst according to any one of [1] to [3], wherein the element X is at least one selected from the group consisting of metals of Group 4 of the Periodic Table.
[5] The catalyst according to any one of [1] to [4], wherein the element X is at least one selected from Hf and Zr.
[6] The catalyst according to any one of [1] to [5], wherein the element Z is Si.
[7] The catalyst according to any one of [1] to [6], wherein the flaky particle has an average aspect ratio of 2.5 or more.
[8] The catalyst according to any one of [1] to [7], wherein the catalyst is a diene compound synthesis catalyst for synthesizing a diene compound from a raw material gas including an alcohol.
[9] The catalyst according to [8], wherein the alcohol is ethanol and the diene compound is a conjugated diene compound.
[10] The catalyst according to any one of [1] to [9], wherein the catalyst forms a secondary particle composed of primary particles being the flaky particle.
[11] The catalyst according to any one of [1] to [10], wherein the pores have an average pore length of 50 to 600 nm.
[12] A method for producing a diene compound, comprising producing a diene compound from a raw material gas including an alcohol by using the catalyst according to any one of [1] to [11].
[13] The method for producing the diene compound according to [12], wherein the alcohol is ethanol and the diene compound is a conjugated diene compound.
[14] The method for producing the diene compound according to [13], wherein the conjugated diene compound is 1,3-butadiene.
[15] A method for producing styrene-butadiene rubber (SBR) or butadiene rubber (BR) using the 1,3-butadiene according to [14] as a raw material gas.

### Advantageous Effects of Invention

According to the catalyst of the present invention, an overreaction to a polymer can be suppressed and a diene compound, particularly butadiene, can be produced at a high yield. Further, by suppressing overreaction, coke deterioration is suppressed, the catalyst life is long, and continuous operation for a long time can be enabled. That is, a catalyst that is capable of efficiently producing butadiene and is less susceptible to a decrease in the butadiene yield even in continuous operation for a long time can be provided.

### Brief Description of Drawings

[Fig. 1] Fig. 1 is a scanning electron microscope (hereinafter, referred to as "SEM") photograph and a transmission electron microscope (hereinafter, referred to as "TEM") photograph of the catalyst according to Example 1 of the present application.
[Fig. 2] Fig. 2 is an SEM photograph and a TEM photograph of the catalyst according to Comparative Example 1 of the present application.
[Fig. 3] Fig. 3 is a schematic diagram illustrating a diene compound production apparatus according to one embodiment of the present invention.

### Description of Embodiment

Hereinafter, a mode of carrying out the present invention will be described in detail. However, the following description is an example of the embodiments of the present invention. The present invention is not limited to this subject matter, and can be carried out by modifying within the gist thereof.

### <Catalyst>

The catalyst according to the present embodiment comprises a flaky particle comprising at least one element X selected from the group consisting of Groups 3 to 6 of the Periodic Table and at least one element Z selected from the group consisting of Group 14 elements, and has pores in a thickness direction of the flaky particle.

The catalyst of the present invention has a flaky catalyst particle shape, and therefore pores can be formed in the thickness direction of the flake shapes. Moreover, the pore length depends on the thickness, and therefore the pore length can be controlled by controlling the thickness of the flake shapes. The inside of the pores serves as reaction sites, and therefore the raw material enters the pores, reacts at the active sites, and the product separates from the pores. The catalyst of the present invention can control the pore length to an appropriate length, and therefore the pore length can be controlled so as to be long enough to progress the reaction and not be too long in order to avoid overreaction. Since an appropriate reaction progresses without the progress of overreaction, the yield of the required product can be increased, coke deterioration can be suppressed, and the catalyst life can be extended.

Further, the catalyst of the present invention may have any flake shape. For example, a flaky catalyst can be easily produced by forming the catalyst as the composite oxide described later. The catalyst may be flaky, and the active species may be an element constituting a composite oxide, or may form a flaky carrier that supports the active species thereon. The catalyst of the present invention is preferably a composite oxide in which the active species is arranged in the skeleton because the dispersibility of the active species is high and aggregation is unlikely to occur.

As described above, the catalyst of the present invention has a flake shape, and therefore the pores can be controlled and the active species can be highly dispersed, so that a good yield and a long catalyst life can be achieved.

Hereinafter, each constituent component will be described in detail.

### (Constituent elements X and Z of the catalyst)

Examples of the element X include Group 3 elements such as scandium (Sc), yttrium (Y), lanthanum (La), and cerium (Ce); Group 4 elements such as titanium (Ti), zirconium (Zr), and hafnium (Hf); Group 5 elements such as vanadium (V), niobium (Nb), and tantalum (Ta); and Group 6 elements such as chromium (Cr), molybdenum (Mo), and tungsten (W). Of these, the element X is preferably a Group 3 element, a Group 4 element, or a Group 5 element, more preferably a Group 4 element or a Group 5 element, and further preferably a Group 4 element. As the Group 4 element, Ti, Zr and Hf are preferable, and as the Group 5 element, Nb and Ta are preferable. In particular, among the elements of Group 4, Zr and Hf are preferable.

The catalyst may have one type of these elements alone or may have a plurality of two or more types of these elements. Further, when the catalyst has a plurality of elements, the elements may be a plurality of elements in the same group, or may be a combination with an element of another group. For example, among the elements of Group 4, the catalyst may have a combination of Zr and Hf, or a combination of Group 4 Hf and Group 5 Ta or Nb.

In the present invention, the combination of Hf and Zr is particularly preferable, and with this combination, particularly excellent activity and catalyst life can be obtained. It is considered that Zr has an effect of making the catalyst flaky when forming the composite oxide, and Hf functions as the active species of the catalyst.

The element Z is an element selected from Group 14 elements. Specific examples include carbon (C), silicon (Si), germanium (Ge), tin (Sn), and the like.

Of these, the element Z is preferably C or Si, and more preferably Si.

One type of the element Z may be included, or two or more types may be included in combination.

A particularly preferable combination of the elements X and Z is Hf and Zr as the element X and Si as the element Z.

The molar content ({X / (X + Z)} × 100) of the element X with respect to the total amount (moles) of the element X and the element Z in the catalyst is preferably 1.0 to 10.0 mol%, more preferably 2.0 to 9.0 mol%, and further preferably 5.0 to 6.0 mol%.

When two or more of the element X are included in combination and two or more of the element Z are included in combination, the molar content is calculated based on the sum of those two or more of the element X and two or more of the element Z.

### (Catalyst structure)

The catalyst of the present invention is characterized by having a flake shape, and the structure of the catalyst is not particularly limited as long as the catalyst has a flake shape.

An example of a method for forming the catalyst into a flake shape includes forming a composite oxide with the elements X and Z.

As used herein, "composite oxide" refers to an oxide in which two or more elements (metals, etc.) comprising the element X and the element Z coexist. It is preferable that the composite oxide has a crystal structure in which the element X and the element Z are bonded via an oxygen. Here, it is presumed that the element Z is the main component forming the skeleton of the catalyst according to the present invention, and that some of the element Z is replaced with the element X. The element X is the active species of the present catalyst. The element X has a very high dispersion because it forms a part of the skeleton of the composite oxide, and does not aggregate.

Further, for example, a supported catalyst in which the element X is supported on the surface of the carrier containing the element Z is not included in the "composite oxide" described above, but a catalyst in which a flaky carrier containing the element Z is formed and the element X is supported thereon is one mode of the present invention as long as it has a flake shape.

To the extent that the effects of the present invention are not impaired, the catalyst of the present invention may comprise elements other than the element X and the element Z, such as zinc (Zn), as constituent elements of the composite oxide.

In addition, when elements other than the element X and the element Z are included as the constituent elements of the composite oxide (hereinafter, such elements are also referred to as "other elements"), regarding the molar ratio of the element X, the element Z, and the other elements, it is preferable that the molar ratio of the element Z is reduced by the molar ratio of the other elements, as described above. In other words, the molar ratio of the element X is preferably the same molar ratio regardless of whether or not other elements are included. This is the same even when two or more other elements are included.

### (Catalyst shape)

The catalyst of the present invention is characterized in that the catalyst shape is flaky and the catalyst has pores in the thickness direction.

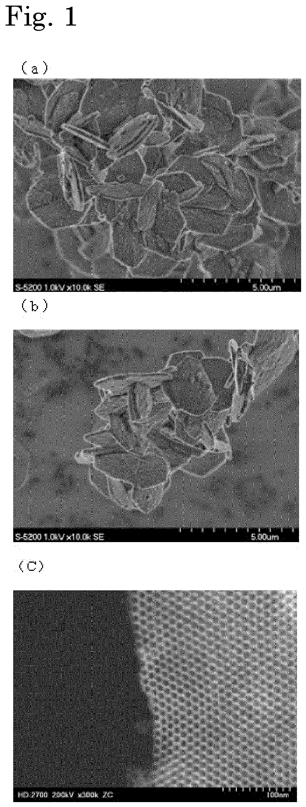

As used herein, "flaky" is a thin shape, and refers to a structure having a very thin thickness with respect to the diameter of a plane as shown in Figs. 1(a) and 1(b), for example. The average aspect ratio (longest diameter / thickness in the plane direction) is preferably 2.5 or more, more preferably 5 or more, and further preferably 10 or more. There is no particular limit on the upper limit of the average aspect ratio, and it is preferably 50 or less.

The average aspect ratio can be calculated by statistically processing with a scanning electron micrograph (SEM) as illustrated in Figs. 1(a) and 1(b).

Since the catalyst of the present invention is flaky, the dispersibility of the active species is high and the frequency of contact with the raw material is increased, and therefore the activity is high. In addition, since the active species is uniformly dispersed, the reaction progresses uniformly, overreaction is suppressed, and coke generation is suppressed. Therefore, it is considered that a high yield is exhibited and the catalyst life is very long.

The average thickness of the catalyst (flaky particle) of the present invention is preferably 50 to 600 nm. The range of the thickness is an important factor for controlling the pore length of the pores in the thickness direction, and the pore length described later is linked to the thickness. Since the pore length is preferably 50 to 600 nm as described later, the thickness of the catalyst is also preferably 50 nm to 600 nm, more preferably 75 to 400 nm, and further preferably 100 to 200 nm.

The average thickness of the flaky particle can be measured by the method described in the examples.

Further, in the catalyst of the present invention, as illustrated in Figs. 1(a) and 1(b), it is preferable that flaky primary particles gather to some extent to form a secondary particle. Dispersion in the form of primary particles is difficult, while a secondary particle is stable.

### (Pores)

Fig. 1(c) illustrates a TEM photograph of the pores of the present invention. It can be seen that the pores are lined up in the thickness direction of the flake.

The pores in the thickness direction of the flaky particle in the catalyst of the present invention preferably have a pore length of 50 to 600 nm. Since the inside of the pores is the reaction site of the present catalyst, it is considered that the reaction progresses in the process of the raw material passing through the pores. Therefore, in order to allow the reaction to progress sufficiently, it is preferable that the pore length is 50 nm or more. From the above viewpoint, the pore length is more preferably 75 nm or more, and further preferably 100 nm or more.

On the other hand, if the pore length is too long, there is a possibility of overreaction progressing, polymer formation as well as coking occur, leading to deterioration of the catalyst. When the pore length is 600 nm or less, the progress of such overreaction can be suppressed. From the above viewpoint, the pore length is more preferably 400 nm or less, and further preferably 200 nm or less.

The "polymer" produced by the overreaction refers to unsaturated carbides and saturated carbides having five or more carbon atoms, such as pentadiene, pentene, pentane, hexadiene, hexatriene, hexene, and hexane.

Further, the pores of the catalyst according to the present invention are preferably mesopores. By having mesopores, the diffusibility of the raw material gas (alcohol, etc.) into the catalyst is improved, and the contact area between the raw material gas and the catalyst increases. As a result, even when the alcohol concentration is high, the raw material conversion rate and diene compound selectivity are improved. The catalyst has an average pore size of 2 to 50 nm, preferably 2 to 30 nm, more preferably 2 to 20 nm, and further preferably 2 to 15 nm. Here, the "average pore size" of the catalyst is a value measured by the following method. That is, the average pore size is calculated from the total pore volume (sum of the pore volume of the catalyst) and the BET specific surface area.

Specifically, the average pore size can be calculated by assuming that the pores have a cylindrical shape (BJH method). The average pore size can then be calculated by 4V1 / A1, in which a BET specific surface area A1 is taken to be the surface area of the side of the cylinder and a total pore volume V1 is taken to be the volume of the cylinder.

The total pore volume of the catalyst is preferably 0.1 to 10.0 mL/g, more preferably 0.1 to 5.0 mL/g, and further preferably 0.1 to 2.0 mL/g. If the total pore volume is 0.1 mL/g or more, the diffusibility of the raw material gas including an alcohol is improved, and the raw material conversion rate and diene compound selectivity are further increased. On the other hand, if the total pore volume is 10.0 mL/g or less, the contact area between the alcohol and the catalyst increases, and the raw material conversion rate and diene compound selectivity are further increased. As used herein, the "total pore volume" of the catalyst is a value measured by gas adsorption. At such time, gas adsorption refers to a method in which nitrogen gas is used as an adsorption gas, nitrogen molecules are adsorbed on the surface of the catalyst, and the pore distribution is measured from the condensation of the molecules.

The specific surface area of the catalyst is preferably 100 to 10000 m²/g, more preferably 200 to 5000 m²/g, further preferably 200 to 1500 m²/g, and particularly preferably 700 to 1200 m²/g.

If the specific surface area is 100 m²/g or more, there are sufficient numbers of active points on the catalyst surface, so that the raw material conversion rate and diene compound selectivity are further increased. As a result, the raw material conversion rate increases even if the content of the raw material is a high concentration with respect to 100% by volume (gas equivalent) of the raw material gas, and for example, a high raw material conversion rate is exhibited even at 100% by volume. On the other hand, if the specific surface area is 10000 m²/g or less, the contact area between the raw material gas and the catalyst increases, and the raw material conversion rate and diene compound selectivity are further increased.

As used herein, "specific surface area" means the BET specific surface area measured by BET gas adsorption with nitrogen as the adsorption gas.

The product of the total pore volume and the specific surface area of the catalyst is preferably 10 to 100000 mL·m²/g², more preferably 20 to 25000 mL·m²/g², and further preferably 20 to 2000 mL·m²/g². If the product is 10 mL·m²/g² or more, there are sufficient numbers of active points on the catalyst surface and the diffusibility of the raw material gas including an alcohol is improved, so that the raw material conversion rate and diene compound selectivity are further increased. On the other hand, if the product is 100000 mL·m²/g² or less, the contact area between the raw material gas and the catalyst tends to be sufficient, and the raw material conversion rate and diene compound selectivity are further increased.

The mesopore volume ratio ((total mesopore volume / total pore volume) × 100) of the catalyst is preferably 50% or more, more preferably 50 to 100%, further preferably 80 to 100%, and particularly preferably 90 to 100%. If the mesopore volume ratio is 50% or more, sufficient mesopores are present in the catalyst, and the diffusibility of the raw material gas including an alcohol is improved, so that the raw material conversion rate and diene compound selectivity are further increased.

The mesopore volume ratio can be controlled based on the usage ratio of the raw material (compound including X, compound including Z, etc.) in the production method described later, firing temperature in the firing step, and the like.

The shape of the mesopores of the catalyst and whether the pore wall forming the mesopores has a crystal structure can be confirmed by observing the diffraction peaks by X-ray diffraction. Specifically, when the pore wall forming the mesopores has a crystal structure, it is preferable that a peak derived from the periodic structure of the mesopores is observed in a low angle range of θ = 6° or less (preferably 0.1° to 6°, and more preferably 0.1° to 1°) by X-ray diffraction.

Specifically, it is preferable that at least one diffraction peak is observed in a low angle range of θ = 6° or less in the X-ray diffraction profile observed using X-ray diffraction, and that the ratio (I / H) of the peak intensity I of the at least one diffraction peak to the width at half maximum H of the diffraction peak is 5000 or more.

Further, the shape and regularity of the mesopores can be confirmed by observing the catalyst with a transmission electron microscope (TEM).

### <Catalyst applications>

The above-described catalyst is preferably a catalyst for synthesizing a diene compound that synthesizes a diene compound from a raw material gas including an alcohol. In such a case, as described later, it is preferable to include ethanol as the alcohol, and it is more preferable to be ethanol. Further, as the diene compound, a conjugated diene compound is preferable, and 1,3-butadiene is particularly preferable, as will be described later.

### <Catalyst production method>

The catalyst according to the present embodiment is not particularly limited as long as it is a method capable of producing a particle comprising the element X and the element Z and having a flake shape. For example, a composite oxide composed of the element X and the element Z in a specific ratio has a flake shape. Examples of the method for producing such a composite oxide include a method for producing such a composite oxide via a solid colloid preparation step and a firing step. Hereinafter, each step will be described.

### [Solid colloid preparation step]

The solid colloid preparation step is s step of obtaining a solid colloid by distilling off at least a part of the solvent in a raw material solution which includes a compound including at least one element X selected from the group consisting of Groups 3 to 6 of the Periodic Table, a compound including at least one element Z selected from the group consisting of Group 14 elements, a surfactant, and a solvent including water.

### (Raw material solution)

The raw material solution includes a compound including at least one element X selected from the group consisting of Groups 3 to 6 of the Periodic Table, a compound including at least one element Z selected from the group consisting of Group 14 elements, a surfactant, and a solvent including water. The raw material solution may further include a compound including another element (e.g., a compound including zinc), an acidic solution, a basic solution, and the like.

Regarding the amounts of the elements X and Z, the ratio of any one of the types of the element X to the element Z is preferably 1/100 to 9/100 in terms of molar ratio, more preferably is 2/100 to 7/100, and further preferably 3/100 to 6/100. When two or more types of the element X are used, the second and subsequent types of the element may be added in an arbitrary amount.

As any one of the types of X, Group 4 or Group 5 elements are preferable, Group 4 elements are more preferable, and Zr is further preferable. The second element X is an element different from the above-mentioned any one of the types of elements, and is preferably a Group 4 or Group 5 element, more preferably a Group 4 element, and even more preferably Hf.

The production method of the present invention will be described below.
(1) First, water and an acidic solution or a basic solution are added to a predetermined amount of surfactant, and the surfactant is dissolved by stirring at a predetermined speed (e.g., about 50 to 200 rpm) under ordinary temperature and pressure conditions.

It is preferable to add the acidic solution or basic solution so that the raw material solution to be prepared has an acidity or basicity of 0.001 mol/L to 10 mol/L (preferably 0.01 to 5 mol/L). The acidic solution or basic solution promotes the hydrolysis of precursors such as the compound including the element X and the compound including the element Z. By being in the above range, the hydrolysis rate of the metal precursors is not too high, and as a result, aggregation between single metal oxides is prevented, and the complex oxide can be efficiently obtained.

(2) The compound including the element X is added at a predetermined rate while stirring the aqueous solution in which the surfactant is dissolved at a predetermined speed under ordinary temperature and pressure conditions.

The stirring speed when stirring at the predetermined speed is preferably 10 to 2000 rpm, more preferably 10 to 1000 rpm, and further preferably 10 to 500 rpm. By setting the stirring speed to be within the above range, aggregation is prevented between single metal oxides, and the complex oxide can be efficiently obtained.

Further, the addition rate when adding the compound including the element X at a predetermined rate is preferably 0.1 to 100 mg/min, more preferably 0.1 to 50 mg/min, and further preferably 0.1 to 20 mg/min. In the above range, while being a practical addition rate, aggregation between single metal oxides is prevented, and a complex oxide can be efficiently obtained.

In addition, the concentration of the compound comprising the element X in the raw material solution to be prepared is preferably in the range of 0.0025 to 0.1 mol/L. When the concentration is 0.0025 mol/L or more, a metal (element X) is sufficiently present when the element Z such as a silica raw material is hydrolyzed, and therefore a flaky catalyst is easily formed. On the other hand, when the concentration is 0.1 mol/L or less, aggregation of the metal (element X) is unlikely to occur. From the above viewpoint, the concentration is more preferably 0.005 to 0.05 mol/L, and further preferably 0.01 to 0.025 mol/L.

In the production method according to the present invention, the catalyst of the present invention can be effectively produced by satisfying at least one of the requirements of the ratio of the element X to the element Z, the addition rate of the compound comprising the element X, and the concentration of the compound comprising the element X.

(3) After confirming that all of the compound including the element X has been dissolved, the compound including the element Z is added at a predetermined rate while stirring at a predetermined rate under ordinary temperature and pressure conditions to prepare a raw material solution.

The predetermined rate when adding the compound comprising the element Z is preferably 0.01 to 10 g/min, more preferably 0.01 to 5 g/min, and further preferably 0.01 to 1 g/min. By setting the predetermined rate to be no more than the upper limit value of the above range, the hydrolysis reaction rate of the compound comprising the element Z is prevented from becoming too fast, the reaction progresses more easily around the surfactant, and it is easier to produce mesopores. By setting the predetermined rate to be no less than the lower limit value of the above range, it is possible to prevent hydrolysis with moisture in the air during the addition of the compound comprising the element Z.

The concentration of the compound comprising the element Z in the raw material solution to be prepared is preferably in the range of 0.001 to 1000 g/L, and more preferably in the range of 0.01 to 100 g/L. By setting the concentration to be no more than the upper limit value of the above range, the hydrolysis reaction rate of the compound comprising the element Z is prevented from becoming too fast, the reaction tends to progress around the surfactant, and it is easier to produce mesopores. By setting the concentration to be no less than the lower limit value of the above range, it is possible to prevent hydrolysis with moisture in the air during the addition of the compound comprising the element Z.

In addition, the compound comprising the element Z is added such that a mass ratio to the surfactant (compound comprising the element Z / surfactant) is preferably 0.01 to 100, and more preferably 0.05 to 50, further preferably 0.1 to 10. By adding such that the mass ratio is not more than the upper limit value of the above range, the compound comprising the element Z tends to react around the surfactant and mesopores are formed more easily. By adding such that the mass ratio is not less than the lower limit value of the above range, it is possible to prevent the formation of oxides (silica etc.) partially comprising the element Z due to there being too much surfactant, which results in a divided structure, and mesopores are formed more easily in the catalyst.

The stirring speed of the raw material solution when adding the compound including the element Z is preferably 10 to 2000 rpm, more preferably 10 to 1000 rpm, and further preferably 10 to 500 rpm. By being in the above range, the hydrolysis of the compound including the element Z is not too early, an interaction with the surfactant occurs so that sufficient mesopores are obtained, and the specific surface area can also be increased.

(4) Then, a suspension is obtained by aging the raw material solution.

The suspension becomes a suspension when the compound including the element X and the compound including the element Z in the above-described raw material solution are hydrolyzed and condensed by water, thereby obtaining solids.

As used herein, "aging" means leaving the raw material solution to stand.

At this time, the aging temperature of the raw material solution is preferably 30 to 200°C, and more preferably 35 to 150°C.

Further, the aging time of the raw material solution is preferably 2 hours to 10 days, and more preferably 10 hours to 5 days.

The aging may be performed in two stages. For example, the first aging may be performed by leaving to stand at 30 to 90°C for 1 hour to 5 days, and then a second aging may be performed at a higher temperature than the first aging by leaving to stand at 90°C to 200°C for 1 hour to 5 days.

Hereinafter, the compound including the element X, the compound including the element Z, the compound including zinc, the surfactant, the solvent, the acidic solution, the basic solution, and the like which are used to produce the raw material solution are described.

### (Compound including element X)

Examples of the compound including the element X include, but are not particularly limited to: inorganic salts, such as a chloride, a sulfide, a nitrate, or a carbonate of the element X; organic salts, such as an oxalate, an acetylacetonato salt, a dimethyl glyoxime salt, or an ethylenediamine acetate of the element X; chelate compounds of the element X; carbonyl compounds of the element X; cyclopentadienyl compounds of the element X; ammine complexes of the element X; alkoxide compounds of the element X; alkyl compounds of the element X, and the like.

Specifically, examples include titanium chloride (TiCl₂, TiCl₃, TiCl₄), zirconium chloride (ZrCl₂), hafnium chloride (HfCl₄), niobium chloride (NbCl₅), tantalum chloride (TaCls), vanadium chloride (VCl₃), tungsten chloride (WCl₅), scandium nitrate (Sc(NO₃)₃), yttrium nitrate (Y(NO₃)₃), lanthanum nitrate (La(NO₃)₃), cerium nitrate (Ce(NO₃)₃), and the like.

One type of the above-described compound comprising the element X may be used alone, or two or more types may be used in combination.

The amount of the compound including the element X that is used is, with respect to the total amount (moles) of the compound including the element X and the compound including the element Z, preferably 0.1 to 20 mol%, more preferably 0.5 to 15 mol%, further preferably 0.5 to 6 mol%, and particularly preferably 0.7 to 4 mol%. In addition, when two or more of the compounds including the element X are included in combination, it is preferable that the sum thereof is included in the above range.

### (Compound including element Z)

Examples of the compound including the element Z include, but are not particularly limited to: inorganic salts, such as a chloride, a sulfide, a nitrate, or a carbonate of the element Z; organic salts, such as an oxalate, an acetylacetonato salt, a dimethyl glyoxime salt, or an ethylenediamine acetate of the element Z; chelate compounds of the element Z; carbonyl compounds of the element Z; cyclopentadienyl compounds of the element Z; ammine complexes of the element Z; alkoxide compounds of the element Z; alkyl compounds of the element Z, and the like.

Among these, it is preferable to use an alkoxide compound including silicon.

As the alkoxide compound including silicon, a compound represented by the following general formula (1) is preferable.

Si(OR)₄ (1)

In formula (1), each R independently represents an alkyl group. The alkyl group is preferably an alkyl group having 1 to 4 carbon atoms, and is more preferably an ethyl group.

Specific examples of the alkoxide compound including silicon include tetramethoxysilane, tetraethoxysilane, tetrapropoxisilane, and the like. Among these, it is preferable to use tetraethoxysilane.

One type of the above-described compound including the element Z may be used alone, or two or more types may be used in combination.

The amount of the compound including the element Z that is used is, with respect to the total amount (moles) of the compound including the element X and the compound including the element Z, preferably 80 to 99.9 mol%, more preferably 85 to 99.5 mol%, and further preferably 94 to 99.5 mol%. In addition, when two or more of the compound including the element Z are included in combination, it is preferable that the sum thereof is included in the above range.

### (Compound including zinc)

Examples of the compound including zinc include, but are not particularly limited to: inorganic salts, such as a chloride, a sulfide, a nitrate, or a carbonate of zinc; organic salts, such as an oxalate, an acetylacetonato salt, a dimethyl glyoxime salt, or an ethylenediamine acetate of zinc; chelate compounds of zinc; carbonyl compounds of zinc; cyclopentadienyl compounds of zinc; ammine complexes of zinc; alkoxide compounds of zinc; alkyl compounds of zinc, and the like.

Specifically, examples include zinc chloride (ZnCl₂), zinc sulfide (ZnS), zinc nitrate (Zn(NO₃)₂), and the like.

One type of the above-described compound including zinc may be used alone, or two or more types may be used in combination.

The amount of the compound including zinc that is used is, with respect to the total amount (moles) of the compound including the element X, the compound including the element Z, and the compound including zinc, preferably 0.1 to 20 mol%, more preferably 0.5 to 15 mol%, and further preferably 0.5 to 6 mol%.

### (Surfactant)

By using a surfactant in the production of the catalyst, a catalyst having mesopores can be obtained. More specifically, micelles are formed through the addition of the surfactant, and the obtained micelles serve as a mold with a precursor of a complex oxide formed on the surface thereof. By performing the firing described later on such a precursor, the surfactant can be removed to produce a catalyst having mesopores. Depending on the density, the shape of the micelles is spherical, cylindrical, lamellar, gyroidal, or vesicle-shaped.

Examples of the surfactant include, but are not particularly limited to, cationic surfactants, non-ionic surfactants, and the like.

Examples of the cationic surfactant include cationic surfactants conventionally used for the synthesis of mesoporous silica, such as MCM-41, SBA-15, FMS-16, and the like.

Examples of the non-ionic surfactant include, but are not particularly limited to, a polyalkylene oxide block copolymer having an alkylene oxide chain as a constituent part, a compound formed into an ether with an alcohol, a phenol, or the like on the end of the block copolymer, and the like.

In addition, one type of the alkylene oxide chain included as a constituent unit may be used alone, or two or more types may be used in combination.

Among these, from the viewpoint of the stability of the crystal structure of the pore wall forming the mesopores of the obtained complex oxide, it is preferable to use a non-ionic surfactant, and it is more preferable to use a polyalkylene oxide block copolymer. From the viewpoint of the stability of the crystal structure of the pore wall forming the mesopores of the obtained complex oxide, it is more preferable to use a polyalkylene oxide block copolymer having a polyethylene oxide chain (CH₂CH₂O)ₘ and a polypropylene oxide chain (CH₂CH(CH₃)O)ₙ as constituent units. Here, m and n are 1 to 1000, preferably m is 1 to 200, and n is 1 to 100. More preferably, m is 1 to 200, n is 1 to 100, and m + n is 2 to 300. An end of the polymer may be formed as an ether with a hydrogen atom, a hydroxyl group, or an alcohol or phenol.

Among the above-described polyalkylene oxide block copolymers, from the viewpoint of the stability of the crystal structure of the pore wall forming the mesopores of the obtained complex oxide, a polyalkylene oxide block copolymer represented by the following general formula (2) is preferable.

HO((CH₂CH₂O)ᵣ(CH₂CH(CH₃)O)ₛ(CH₂CH₂O)ₜ)H (2)

From the viewpoint of forming the above-described preferable average pore size of the synthesis catalyst of the present invention, r is preferably 1 to 100, s is preferably 1 to 100, and t is preferably 1 to 100. Further, r + s + t is preferably 3 to 300.

The method for obtaining the polyalkylene oxide block copolymer is not particularly limited, and a polyalkylene oxide block copolymer produced using a conventionally known production method may be used, or a commercially available product may be used.

Examples of commercially available polyalkylene oxide block copolymers include the product P123 [(HO(CH₂CH₂O)₂₀(CH₂CH(CH₃)O)₇₀(CH₂CH₂O)₂₀)H], the product P85 [(HO(CH₂CH₂O)₂₆(CH₂CH(CH₃)O)₃₉(CH₂CH₂O)₂₆)H], and the product P103 [(HO(CH₂CH₂O)₅₆(CH₂CH(CH₃)O)₁₇(CH₂CH₂O)₅₆H)], which are manufactured by BASF.

One type of the above-described surfactant may be used alone, or two or more types may be used in combination.

The shape and pore diameter of the mesopores can be controlled by appropriately changing the type of above-described surfactant and the like.

The amount of the surfactant that is used is, based on the assumption of attaining the above-described mass ratio between the compound including the element Z and the surfactant (compound including the element Z / surfactant), with respect to 100 parts by mass of solvent, preferably 3 to 20 parts by mass, more preferably 5 to 18 parts by mass, and further preferably 7 to 15 parts by mass. It is preferable that the amount of the surfactant that is used is 3 parts by mass or more because the mesopores can be uniformly formed. On the other hand, it is preferable that the amount of the surfactant that is used is 20 parts by mass or less because the surfactant can be dissolved.

### (Solvent)

The solvent includes water. The solvent may further include an organic solvent.

The water is not particularly limited, but ion-exchanged water from which metal ions and the like have been removed, or distilled water, is preferable.

Examples of the organic solvent include, but are not particularly limited to, aliphatic linear alcohols such as methanol, ethanol, n-propanol, and n-hexanol.

Among these, from the viewpoint of handleability, the organic solvent is preferably methanol or ethanol.

One type of the organic solvent may be used alone, or two or more types may be used in combination.

The amount of water that is used is, with respect to 1 part by mass of the surfactant, preferably 5 to 35 parts by mass, and more preferably 5 to 20 parts by mass. It is preferable that the amount of water that is used is 5 parts by mass or more because the surfactant can be dissolved. On the other hand, it is preferable that the amount of water that is used is 35 parts by mass or less because the mesopores can be uniformly formed.

The amount of water that is used is, with respect to the total amount (moles) of the compound including the element X and the compound including the element Z, preferably 100 mol% to 10000 mol%, and more preferably 1000 to 8000 mol%. It is preferable that the amount of water that is used is 100 mol% or more because hydrolysis can be performed. On the other hand, it is preferable that the amount of water that is used is 10000 mol% or less because the solids do not dissolve.

Further, when an organic solvent that is used is included as the solvent, the amount of the organic solvent is, with respect to the water, preferably 10 to 50% by volume, and more preferably 10 to 25% by volume. It is preferable that the amount of organic solvent that is used is 10% or more by volume because the element X and element Z can be dissolved. On the other hand, it is preferable that the amount of organic solvent that is used is 50% by volume or less because hydrolysis can be performed.

### (Acidic solution)

The acidic solution has the function of promoting the production of solids by hydrolysis, which is described later.

Examples of the acidic solution include, but are not particularly limited to, an aqueous solution in which an inorganic acid such as hydrogen chloride, sulfuric acid, nitric acid, or phosphoric acid is dissolved.

The amount of the acidic solution that is used is an amount by which the number of moles of acid included in the acidic solution becomes, with respect to the total amount (moles) of the compound including the element X and the compound including the element Z, preferably 0.01 to 10.0 mol%, and more preferably 0.1 to 8.0 mol%.

### (Basic solution)

The basic solution has the function of promoting the production of solids by hydrolysis, which is described later. Generally, any one of the above-described acidic solution and a basic solution is used.

Examples of the basic solution include, but are not particularly limited to, an aqueous solution in which an inorganic base such as sodium hydroxide, calcium hydroxide, or ammonia is dissolved.

The amount of the basic solution that is used is an amount that becomes, with respect to the total amount (moles) of the compound including the element X and the compound including the element Z, preferably 0.01 to 10.0 mol%, and more preferably 0.1 to 8.0 mol%.

### (Preparation of solid colloid)

The solid colloid can be obtained by filtering, appropriately washing, and drying the above-described suspension.

By performing a step of preparing a solid colloid in this way, the catalyst can have better uniformity compared with when the suspension is directly fired. As used herein, "solid colloid" means that the amount of solvent contained in the solid colloid is 5% or less of the total volume of the solid colloid.

The drying temperature for obtaining the solid colloid is preferably 20 to 200°C, and more preferably 50 to 150°C.

The drying time is preferably 1 hour to 10 days, and more preferably 2 hours to 5 days. By setting to be in the above ranges, the solvent is removed, a sufficient mesopore diameter can be obtained in the subsequent firing step, and the specific surface area can be increased.

### [Firing step]

The firing step is a step of firing the solid colloid. By firing the solid colloid, the surfactant used as the mold is removed, and the catalyst according to the present embodiment can be produced.

The firing temperature is preferably 200 to 800°C, and more preferably 400 to 600°C. It is preferable that the firing temperature is not less than 200°C because impurities derived from the surfactant do not remain, or hardly remain, in the catalyst. On the other hand, it is preferable that the firing temperature is not more than 800°C because the stability of the crystal structure of the pore wall forming the mesopores of the catalyst can be improved.

Here, the rate of temperature increases up to the firing temperature is preferably 0.1 to 100 °C/min, more preferably 0.5 to 50 °C/ min, and further preferably 1 to 20 °C/min. By setting to be in the above range, there is no temperature difference between the surface and the inside of the formed article, and a sufficient mesoporous diameter can be obtained. As a result, the specific surface area of the catalyst can also be increased.

The firing time is preferably 10 minutes to 2 days, and more preferably 1 to 10 hours. It is preferable that the firing time is not less than 10 minutes because impurities derived from the surfactant do not remain, or hardly remain, in the catalyst. On the other hand, it is preferable that the firing time is not more than 2 days because the stability of the crystal structure of the pore wall forming the mesopores of the catalyst can be improved.

### (Diene compound production apparatus)

The diene compound production apparatus includes a reaction tube filled with the above-described catalyst. By such a production apparatus, a diene compound is produced from the raw material gas including the raw material.

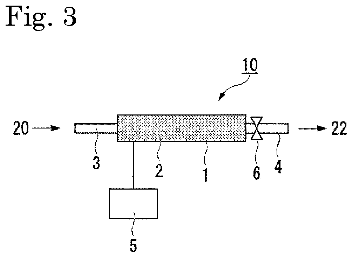

Hereinafter, a butadiene production apparatus, which is an example of the diene compound production apparatus, will be described with reference to Fig. 3.

A butadiene production apparatus 10 (hereinafter, simply referred to as the "production apparatus 10") of the present embodiment includes a reaction tube 1, a feed tube 3, a discharge tube 4, a temperature control unit 5, and a pressure control unit 6.

A reaction bed 2 is included inside the reaction tube 1. The reaction bed 2 is filled with the synthesis catalyst of the present invention. The feed tube 3 is connected to the reaction tube 1. The discharge tube 4 is connected to the reaction tube 1. The temperature control unit 5 is connected to the reaction tube 1. The discharge tube 4 includes the pressure control unit 6.

The reaction bed 2 may have only the catalyst according to the present embodiment, or may have a catalyst other than the catalyst according to the present embodiment together with the catalyst according to the present embodiment. Further, the reaction bed 2 may further have a dilution material. The dilution material prevents the catalyst from excessively generating heat.

The dilution material is, for example, quartz sand, alumina balls, aluminum balls, aluminum shots, and the like.

When the dilution material is filled into the reaction bed 2, the mass ratio represented by the dilution material / synthesis catalyst is determined taking into consideration the type and specific gravity of each of those, and for example, 0.5 to 5 is preferable.

The reaction bed may be a fixed bed, a moving bed, a fluidized bed, or the like.

The reaction tube 1 is preferably composed of a material inert to the raw material gas and the synthesized product. The reaction tube 1 preferably has a shape that is capable of withstanding heating at about 100 to 600°C and pressurization of about 10 MPa. The reaction tube 1 is, for example, a substantially cylindrical member made of stainless steel.

The feed tube 3 is feed means for feeding the raw material gas into the reaction tube 1. The feed tube 3 is, for example, a tube made of stainless steel.

The discharge tube 4 is discharge means for discharging a gas including the product synthesized at the reaction bed 2. The discharge tube 4 is, for example, a tube made of stainless steel.

The temperature control unit 5 may be capable of setting the reaction bed 2 in the reaction tube 1 to any temperature. For example, the temperature control unit 5 controls the temperature of an electric furnace or the like (not shown) provided around the reaction tube 1, and adjusts the reaction bed 2 in the reaction tube 1 to any temperature.

The pressure control unit 6 may be capable of making the pressure in the reaction tube 1 any pressure. The pressure control unit 6 is, for example, a known pressure valve or the like.

The production apparatus 10 may include a known device such as a gas flow control unit that adjusts the flow rate of a gas, such as mass flow.

### <Method for producing diene compound>

According to one mode of the present invention, there is provided a method for producing a diene compound. The method for producing the diene compound includes contacting the catalyst according to the present invention with a raw material gas including an alcohol to produce a diene compound.

### [Catalyst]

As the catalyst, the above-described catalyst is used, and therefore a description thereof is omitted here.

The amount of catalyst that is used is, with respect to the raw material gas, preferably 0.1 to 10 g/g·h, and more preferably 1 to 5 g/g·h. It is preferable that the amount of catalyst used is 0.1 g/g·h or more because the reaction conversion rate can be improved. On the other hand, it is preferable that the amount of catalyst used is 10 g/g·h or less because the generation of by-products can be suppressed.

### [Raw material gas]

The raw material gas includes an alcohol. In addition, the raw material gas may further include an inert gas, and the like.

### (Alcohol)

Examples of the alcohol include, but are not particularly limited to, alcohols having 1 to 6 carbon atoms. Specific examples of the alcohol include methanol, ethanol, propanol, butanol, pentanol, hexanol, and the like.

In principle, the diene compound that is obtained differs depending on the alcohol that is used. For example, when ethanol is used, butadiene is obtained. In addition, when propanol is used, hexadiene is obtained. Furthermore, when butanol is used, octadiene is obtained.

One type of alcohol may be used alone, or two or more types may be used in combination. However, from the viewpoint of suppressing side reactions, it is preferable to use one type of alcohol alone.

The concentration of the alcohol in the raw material gas is, with respect to 100% by volume of the raw material gas, preferably 10% by volume or more, more preferably 15% by volume or more, further preferably 20% by volume or more, and most preferably 30% by volume or more. When two or more types of the alcohol are used in combination, it is preferable that the sum thereof is included in the above range. By using the catalyst according to the present invention, the reaction can be efficiently progressed even when the alcohol concentration in the raw material gas is high.

### (Inert gas)

Examples of the inert gas include, but are not particularly limited to, nitrogen gas, argon gas, and the like. One type of these inert gases may be used alone, or two or more types may be used in combination.

The concentration of the inert gas is, with respect to 100% by volume of the raw material gas, preferably 90% by volume or less, more preferably 30 to 90% by volume, further preferably 50 to 90% by volume, and particularly preferably 60 to 80% by volume.

### [Contact]

The mode of contacting the catalyst with the raw material gas is not particularly limited, but for example, it is preferable to flow the raw material gas over a reaction bed in a reaction tube to contact the raw material gas with the synthesis catalyst in the reaction bed. As described above, the mode of contacting the catalyst is not particularly limited, and for example a fixed bed, a fluidized bed, or the like can be suitably used.

The temperature (reaction temperature) when the raw material gas is contacted with the catalyst is preferably 100 to 600°C, more preferably 200 to 500°C, and further preferably 250 to 450°C. It is preferable that the reaction temperature is 100°C or more because the reaction rate is sufficiently high and the diene compound can be produced more efficiently. On the other hand, it is preferable that the reaction temperature is 600°C or lower because deterioration of the catalyst can be prevented or suppressed.

The pressure (reaction pressure) when the raw material gas is contacted with the catalyst is preferably 0.1 to 10 MPa, and more preferably 0.1 to 3 MPa. It is preferable that the reaction pressure is 0.1 MPa or more because the reaction rate is high and the diene compound can be produced more efficiently. On the other hand, it is preferable that the reaction pressure is 10 MPa or less because deterioration of the catalyst can be prevented or suppressed.

The space velocity (SV) of the raw material gas in the reaction bed is generally adjusted as appropriate in consideration of the reaction pressure and reaction temperature, but in terms of standard state, is preferably set to be 0.1 to 10000 h⁻¹.

For example, when using production apparatus 10 to produce butadiene, the insides of the reaction tube 1 is set to any temperature and any by the temperature control unit 5 and the pressure control unit 6. A gasified raw material gas 20 is supplied from the feed tube 3 into the reaction tube 1. In reaction tube 1, the raw material is contacted with the synthesis catalyst and reacted to produce a diene compound such as butadiene. A production gas 22 including the diene compound such as butadiene is discharged from the discharge tube 4. The production gas 22 may also include compounds such as acetaldehyde, propylene, ethylene, and the like.

The production gas including the diene compound (production gas 22 in Fig. 3) is subjected to purification, such as gas liquid separation or distillation purification, as necessary, and unreacted raw materials and by-products are removed.

Further, the present invention can also produce a diene compound from bioethanol to reduce environmental impact.

### <Method for producing SBR or BR>

The 1,3-butadiene produced by the production method of the present invention can then be suitably used as a raw material for styrene-butadiene rubber (SBR) or butadiene rubber (BR).

### Examples

Hereinafter, the present invention will be specifically described by using examples, but the present invention is not limited to the following description.

### (Evaluation of catalyst physical properties: average diameter (nm) and average thickness (nm) of catalyst)

The average diameter (nm) and average thickness (nm) of the catalyst obtained in each example were measured by scanning electron microscope (SEM) observation. Specifically, three scanning electron microscope (SEM) images (observation images) confirmed as having 20 or more primary particles obtained in each of the examples were arbitrarily selected, 10 particles were randomly selected from each image, and the diameter (nm) and thickness (nm) of the 10 particles were measured. The average diameter (nm) and average thickness (nm) of the primary particles were obtained from the average value of the obtained total data. In cases where the diameters of the selected particles were not uniform, the diameter of a circumscribed circle of the particles was adopted as the diameter of each particle. Further, it was confirmed from the transmission electron microscope (TEM) images that pores were present in the thickness direction.

### (Evaluation of catalyst reaction: synthesis of diene compound)

Using the catalysts prepared in Examples 1 to 3 and Comparative Examples 1 and 2, the yield of 1,3-butadiene (BD) when converting ethanol to 1,3-butadiene was measured after one hour from the start of the reaction and after 20 hours from the start of the reaction, respectively.

Specifically, a reaction bed was formed by filling a stainless-steel cylindrical reaction tube having a diameter of 1/2 inch (1.27cm) and a length of 15.7 inches (40 cm) with 3.4 g of the catalyst. Next, the reaction temperature (temperature of the reaction bed) was set to 325°C, and the reaction pressure (pressure of the reaction bed) was set to 0.1 MPa. The raw material gas was fed into the reaction tube at SV 1200 L / hr / catalyst amount (L-catalyst) to obtain a production gas. The raw material gas was a mixed gas of 30% by volume ethanol (gas equivalent) and 70% by volume nitrogen (gas equivalent).

The recovered production gas was analyzed by gas chromatography to determine the BD yield ([conversion rate] × [BD selectivity]). The "BD selectivity" is, of the number of moles of the raw material consumed by the reaction using the catalyst, the percentage of the number of moles of the raw material converted to butadiene. In addition, the "conversion rate (raw material conversion rate)" is the percentage of the number of moles consumed among the number of moles of the raw material included in the raw material gas.

The evaluation of the catalyst was performed based on the yield of butadiene. The higher the yield, the better the catalytic activity. Further, the difference obtained by subtracting the yield after one hour from the start of the reaction from the yield after 20 hours from the start of the reaction was taken as the amount of change in yield (%). The closer this value is to 0, the more the decrease in yield with respect to the elapsed time is suppressed, and the longer the catalyst life is.

### Example 1

### A beaker was charged with 2 g of P123

([(HO(CH₂CH₂O)₂₀(CH₂CH(CH₃)O)₇₀(CH₂CH₂O)₂₀)H], manufactured by BASF) as a surfactant, then 65 mL of water and 35 mL of 2 N hydrochloric acid were added, and the mixture was stirred at a speed of 100 rpm under ordinary temperature and pressure conditions to dissolve the surfactant. While stirring the aqueous solution in which the surfactant was dissolved at a rate of 100 rpm under ordinary temperature and pressure conditions, 64 mg of hafnium chloride (HfCl₄) and 225 mg of zirconium chloride (ZrCl₄) were added in that order at a rate of 10 mg/min. After visually confirming that all of the hafnium chloride and zirconium chloride had dissolved, the mixture was stirred at a rate of 100 rpm under ordinary temperature and pressure conditions, and 4.2 g of tetraethoxysilane was added at a rate of addition of 0.1 g/min to prepare a raw material solution. The concentration of hafnium chloride in the raw material solution was 0.64 g/L, the zirconium chloride concentration was 2.25 g/L, the tetraethoxysilane concentration was 4.8 g/L, and the hydrochloric acid concentration was 0.7 mol/L. Then, a suspension was obtained by leaving the raw material solution to stand for 20 hours at 40°C.

The suspension was left to stand for 20 hours at 100°C, then filtered, and washed with ethanol and water. The powder was then transferred to a petri dish, and dried for 4 hours in an oven kept at a temperature of 110°C to obtain a solid colloid.

The obtained solid colloid was heated to 550°C at a rate of temperature increase of 5 °C/min in an air atmosphere using an electric furnace, and fired at 550°C for 5 hours to produce a catalyst, which was a composite oxide including Hf, Zr, and Si.

The obtained catalyst formed a composite oxide and was flaky.

The above-described reaction evaluation was carried out on the catalyst, and the BD yield one hour after the start of the reaction and 20 hours after the start of the reaction were each determined. The results are shown in Table 1.

### Example 2

Catalyst production and catalyst evaluation were carried out in the same manner as in Example 1, except that the amount of zirconium chloride (ZrCl₄) used in Example 1 was changed to 117 mg. The results are shown in Table 1. The catalyst formed a composite oxide and was flaky.

### Example 3

Catalyst production and catalyst evaluation were carried out in the same manner as in Example 1, except that the amount of zirconium chloride (ZrCl₄) used in Example 1 was changed to 78 mg. The results are shown in Table 1. The catalyst formed a composite oxide and was flaky.

### Comparative Example 1

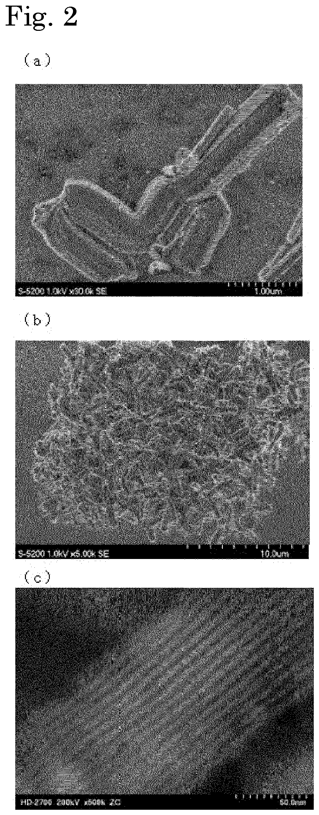

A catalyst, which is a composite oxide including Hf and Si, was produced and evaluated in the same manner as in Example 1, except that zirconium chloride (ZrCl₄) was not used. The results are shown in Table 1. Although the catalyst formed a composite oxide, its shape was tubular as illustrated in Figs. 2(a) and 2(b). Further, as is clear from the TEM photograph of Fig. 2C, pores were formed in the length direction of the tube, and it can be seen that the direction of the pores is completely different from that of the catalyst of the present invention.

### Comparative Example 2

Catalyst production was carried out in the same manner as in Example 1, except that the amount of zirconium chloride (ZrCl₄) used in Example 1 was changed to 466 mg. The catalyst did not form crystals, and was an irregular shape. The evaluation results of the catalyst are shown in Table 1.

**[Table 1]**

| | Element X | Element Z | X/Z (molar ratio) | (X/(X+Z)) × 100 (mol%) | Shape | Average Diameter (nm) | Average Thickness (nm) | Average Aspect Ratio | Butadiene Yield (%) | | Amount of Change in Yield (%) |
|---|---|---|---|---|---|---|---|---|---|---|---|
| | Type | Type | | | | | | | After 1 hour | After 20 hours | |
| Example 1 | Hf | Si | 1/100 | 5.7 | flaky | 2448 | 153 | 16.0 | 51.92 | 48.38 | -3.54 |
| | Zr | | 5/100 | | | | | | | | |
| Example 2 | Hf | Si | 1/100 | 3.4 | flaky | 1860 | 269 | 6.93 | 53.63 | 46.18 | -7.45 |
| | Zr | | 2.5/100 | | | | | | | | |
| Example 3 | Hf | Si | 1/100 | 2.6 | flaky | 1187 | 403 | 2.95 | 51.22 | 45.53 | -5.69 |
| | Zr | | 1.67/100 | | | | | | | | |
| Comparative Example 1 | Hf | Si | 1/100 | 1.0 | tubular | - | - | - | 54.47 | 31.42 | -23.05 |
| Comparative Example 2 | Hf | Si | 1/100 | 9.9 | irregular | - | - | - | 38.03 | 20.02 | -18.01 |
| | Zr | | 10/100 | | | | | | | | |

From the above results, it can be seen that the catalysts of Examples 1 to 3, which are flaky, have a very small amount of change in yield and a long catalyst life. It is considered that this is due to the catalyst of the present invention being flaky, and therefore the pore length of the pores, which are thought to be the main reaction site in the thickness direction, can be controlled. Since the pore length is appropriate, the raw material such as ethanol reacts in the pores to produce butadiene or the like, which can then rapidly separate from the pores. As a result, overreaction to a polymer is suppressed, and coking, which is the main cause of catalyst deterioration, is suppressed.

In contrast, the catalysts of Comparative Examples 1 and 2 were not flaky, and exhibited a tube shape as illustrated in Fig. 2. It was found that these catalysts had a large amount of change in yield, and that the catalysts deteriorated significantly with the passage of reaction time as compared with the catalysts of the present invention.

### Reference Signs List

- 1: reaction tube
- 2: reaction bed
- 3: feed tube
- 4: discharge tube
- 5: temperature control unit
- 6: pressure control unit
- 10: butadiene production apparatus
- 20: raw material gas
- 30: production gas

## Claims

1. A catalyst comprising a flaky particle comprising:
at least one element X selected from the group consisting of elements in Groups 3 to 6 of the Periodic Table; and
at least one element Z selected from the group consisting of elements in Group 14 of the Periodic Table, wherein
the catalyst has pores in a thickness direction of the flaky particle.

2. The catalyst according to claim 1, wherein the flaky particle has an average thickness of 50 to 600 nm.

3. The catalyst according to claim 1 or 2, wherein the flaky particle is a composite oxide.

4. The catalyst according to any one of claims 1 to 3, wherein the element X is at least one selected from the group consisting of metals of Group 4 of the Periodic Table.

5. The catalyst according to any one of claims 1 to 4, wherein the element X is at least one selected from Hf and Zr.

6. The catalyst according to any one of claims 1 to 5, wherein the element Z is Si.

7. The catalyst according to any one of claims 1 to 6, wherein the flaky particle has an average aspect ratio of 2.5 or more.

8. The catalyst according to any one of claims 1 to 7, wherein the catalyst is a diene compound synthesis catalyst for synthesizing a diene compound from a raw material gas including an alcohol.

9. The catalyst according to claim 8, wherein the alcohol is ethanol and the diene compound is a conjugated diene compound.

10. The catalyst according to any one of claims 1 to 9, wherein the catalyst forms a secondary particle composed of primary particles being the flaky particle.

11. The catalyst according to any one of claims 1 to 10, wherein the pores have an average pore length of 50 to 600 nm.

12. A method for producing a diene compound, comprising producing a diene compound from a raw material gas including an alcohol by using the catalyst according to any one of claims 1 to 11.

13. The method for producing the diene compound according to claim 12, wherein the alcohol is ethanol and the diene compound is a conjugated diene compound.

14. The method for producing the diene compound according to claim 13, wherein the conjugated diene compound is 1,3-butadiene.

15. A method for producing styrene-butadiene rubber (SBR) or butadiene rubber (BR) using the 1,3-butadiene according to claim 14 as a raw material gas.
